# EUROPEAN PATENT APPLICATION

(11) **EP 1 623 969 A1**
(43) Date of publication of application: **08.02.2006**
(21) Application number: 04723004.0
(22) Date of filing: 24.03.2004
(51) Int. Cl.: C07C 57/54, C07C 51/12

(54) **ALPHA-PENTAFLUOROETHYLACRYLIC ACID DERIVATIVE AND PROCESS FOR PRODUCING THE SAME**

(30) Priority: 26.03.2003 JP 2003085170
(71) Applicant: Tosoh F- Tech Inc., Shunan-shi, Yamaguchi 746-0006 (JP)
(72) Inventor: TOKUHISA, Kenji, 7460012 (JP); MIMURA, Hideyuki, 7460011 (JP); KAWADA, Kosuke, 7440063 (JP); ARAI, Shoji, 7450816 (JP)
(74) Representative: Lambert, Ian Robert
(86) International application number: PCT/JP2004/004026
(87) International publication number: WO 2004/085372

(57) **Abstract**

An α-pentafluoroethyl acrylic acid derivative represented by the general formula [I]: 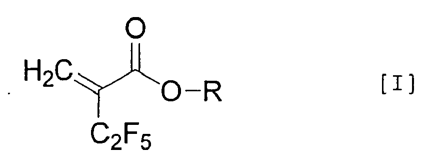
[wherein R represents a hydrogen atom, a non-substituted or substituted aromatic ring, or a straight or branched alkyl group having 1 to 20 carbon(s) which may have a cyclic moiety optionally substituted with at least one substituent (halogen atom, hydroxyl group, straight or branched alkoxy group having 1 to 10 carbon(s) which may have a cyclic moiety, non-substituted or substituted aromatic group)].

## Description

### TECHNICAL FIELD

The present invention relates to an industrially advantageous novel method of producing an α-pentafluoroethyl acrylic acid derivative which is useful as an intermediate for pharmaceuticals or pesticides and as a functional polymer material.

### BACKGROUND ART

There are several reports regarding a method of producing an acrylic acid which is perfluoroalkylated at its α position and an ester thereof. Examples of such production methods include a production method in which 1-perfluoroalkyl-1-halogenoethylene is carbonylated in the presence of a base and a palladium catalyst (Patent document 1); a production method in which 1-perfluoroalkyl-1-halogenoethylene is made into lithio by using n-butyl lithium and caused to react with carbon dioxide (Non-patent document 1); a production method in which a Grignard reagent is synthesized from 1-perfluoroalkyl-1-halogeno ethylene and caused to react with carbon dioxide (Non-patent document 2), and a production method in which zinc, 1-perfluoroalkyl-1-halogeno ethylene and carbon dioxide are caused to react with each other (Patent document 2, Patent document 3).

However, most of the above methods are production methods regarding α-trifluoromethyl acrylic acid derivative. Regarding other perfluoroalkyl groups, only known method is to use zinc for an acrylic acid that is substituted with a perfluorobutyl group. In brief, α-pentafluoroethyl acrylic acid derivatives have not been known, and hence a production method thereof has not been known. Since variation in length of perfluoroalkyl group will result in different affinity and reactivity with solvents and other compounds, there is need to develop a novel production method of α-pentafluoroethyl acrylic acid derivatives.
(Patent document 1) Japanese Patent Laid-Open Publication No. Sho 58-154529
(Patent document 2) Japanese Patent Laid-Open Publication No. Sho 62-129242
(Patent document 3) Japanese Patent Laid-Open Publication No.-2001-288138
(Non-patent document 1) J. Org. Chem., Vol.33, No.1, p.280 (1968)
(Non-patent document 2) J. Fluorine. Chem., Vol.29, p.431 (1985)

### DISCLOSURE OF THE INVENTION

The present invention was devised in consideration of the above technical problems. It is an object of the present invention to provide an α-pentafluoroethyl acrylic acid derivative and a method of producing the same.

The inventors of the present inventions made diligent efforts for solving the above problems and finally accomplished the present invention.

Specifically, the present invention is directed to an α-pentafluoroethyl acrylic acid derivative represented by the general formula [I]: 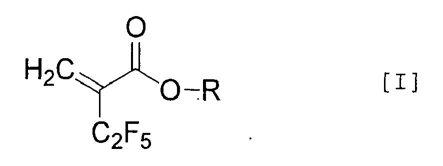
[wherein R represents a hydrogen atom, a non-substituted or substituted aromatic ring, or a straight or branched alkyl group having 1 to 20 carbon(s) which may have a cyclic moiety optionally substituted with at least one substituent (halogen atom, hydroxyl group, straight or branched alkoxy group having 1 to 10 carbon(s) which may have a cyclic moiety, non-substituted or substituted aromatic group)].

In addition, the present invention is directed to a method of producing an α-pentafluoroethyl acrylic acid derivative represented by the general formula [I]: 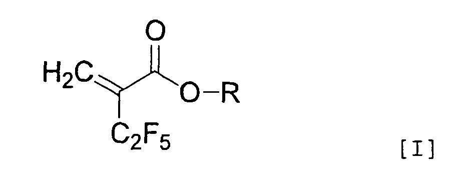
[wherein R represents a hydrogen atom, a non-substituted or substituted aromatic ring, or a straight or branched alkyl group having 1 to 20 carbon(s) which may have a cyclic moiety optionally substituted with at least one substituent (halogen atom, hydroxyl group, straight or branched alkoxy group having 1 to 10 carbon(s) which may have a cyclic moiety, non-substituted or substituted aromatic group)], by letting a hydrocarbon halide represented by the general formula [II]: 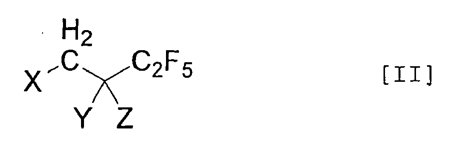
[wherein X represents a halogen atom or forms a bound together with Y, Y represents a hydrogen atom or forms a bond together with X, and Z represents a halogen atom] react with water and an alcohol represented by the general formula [III]:

R-OH [III]

[wherein R is as defined above] in the presence of a palladium catalyst, carbon monoxide, and a base.

### BEST MODE FOR CARRYING OUT THE INVENTION

The present invention will now be described in more detail.

In the general formulae [I] and [III] of the present invention, among the non-substituted or substituted aromatic rings, examples of the non-substituted aromatic rings include phenyl group, naphthyl group, furyl group, thienyl group, pyrrolyl group, pyrazolyl group, oxazolyl group, isoxazolyl group, thiazolyl group, isothiazolyl group, 1,2,5-thiadiazolyl group, 1,2,4-thiadiazolyl group, and pyridyl group, with phenyl group and naphthyl group being preferred. The substituted aromatic rings include aromatic rings in which the above non-substituted aromatic rings are substituted with at least one same or different substituents, and examples of the substituents include lower alkyl groups having 1 to 5 carbon(s) such as methyl group, ethyl group, isopropyl group, and t-butyl group; halogen atoms such as fluorine, chlorine, bromine, and iodine; lower alkoxy groups having 1 to 5 carbon(s) such as hydroxyl group, methoxy group, ethoxy group, isopropyloxy group, and t-butoxy group, and preferably toryl group, mesityl group, and anisyl group. Examples of the halogen atom include fluorine, chlorine, bromine, and iodine. Examples of the straight or branched alkoxy group having 1 to 10 carbon(s) which may have a cyclic moiety include methoxy group, ethoxy group, isopropoxy group, t-butoxy group, cyclopropyloxy group, and cyclohexyloxy group. Examples of the straight or branched alkyl group having 1 to 20 carbon(s) which may have a cyclic moiety include methyl group, ethyl group, propyl group, isopropyl group, butyl group, t-butyl group, 1-methylpropyl group, 2-methylpropyl group, pentyl group, 1,1-dimetylpropyl group, 1,2-dimethylpropyl group, 2,2-dimethylpropyl group, 1-methylbutyl group, 2-methylbutyl group, 3-methylbutyl group, cyclopropyl group; cyclobutyl group, dimethylcyclopropyl group, methylcyclobutyl group, cyclopentyl group, hexyl group, cyclohexyl group, 3-methylcyclohexyl group, 4-methylcyclohexyl group, heptyl group, octyl group, cyclooctyl group, nonyl group, norbornyl group, adamantyl group, bicyclo[2,2,2]octyl group, and bicyclo[3,2,1]octyl group.

In the general formula [II] of the present invention, examples of the halogen atom include fluorine, chlorine, bromine, and iodine, with bromine being preferred. Examples of the palladium catalyst that can be used include metal palladiums such as palladium black and palladium sponge; supported palladium such as palladium/carbon, palladium/alumina, palladium/asbestos, palladium/barium sulfate, palladium/barium carbonate, palladium/calcium carbonate, and palladium/polyethylene amine; palladium salts such as palladium chloride, palladium bromide, palladium iodide, palladium acetate, palladium trifluoroacetate, palladium nitrate, palladium oxide, palladium sulfate, palladium cyanate, allyl palladium chloride dimmer, and palladium acetyl acetate; palladium complex salts and complex compounds such as sodium hexachloro palladate, potassium hexachloro palladate, sodium tetrachloro palladate, potassium tetrachloro palladate, potassium tetrabromo palladate, tetra(acetonitrile)palladium fluoroborate, ammonium tetrachloro palladate, ammonium hexachloro palladate, dichloro bis(acetonitrile)palladium, and dichloro bis(benzonitrile)palladium; amine-based complexes such as dichloroamine palladium, palladium tetraamine nitrate, tetraamine palladium tetrachloro palladate, dichlorodipyridine palladium, dichloro(2,2'-bipyridyl)palladium, dichloro(4,4'-dimethyl-2,2'-bipyridyl)palladium, dichloro(phenanthroline)palladium, (phenanthroline)palladium nitrate, dichloro(tetramethyl phenanthroline)palladium, (tetramethyl phenanthroline)palladium nitrate, diphenanthroline palladium nitrate, and bis(tetramethyl phenanthroline) palladium nitrate; phosphine-based complexes such as dichloro bis(triphenylphosphine)palladium, dichloro bis(tricyclohexylphosphine)palladium, tetrakis (triphenylphosphine)palladium, dichloro[1,2-bis(diphenylphosphino)ethane]palladium, dichloro[1,3-bis(diphenylphosphino)propane]palladium, dichloro[1,4-bis(diphenylphosphino)butane]palladium, and dichloro[1,1'-bis(diphenylphosphino)ferrocene]palladium.

In the cases of amine-based palladium complexes or phosphine-based palladium complexes, they may be prepared in a reaction system by adding a compound which can be a ligand with a palladium compound as a raw material. Examples of the palladium compound to be used in the reaction system, include supported palladium described above, and palladium salts. Examples of the ligand for an amine-based complex that can be used for preparation in a reaction system include ammonia, diethylamine, triethylamine, 1,2-bis(dimethylamino)ethane, 1,2-bis(diphenyamino)ethane, 1,2-bis(dimethylamino)propane, 1,3-bis(dimethylamino)propane, pyridine, aminopyridine, dimethylaminopyridine, 2,2'-bipyridyl, 4,4'-dimethyl-2,2'-bipyridyl, 2,2'-biquinoline, phenanthroline, tetramethyl phenanthroline, and the like. Examples of the ligand for phosphine-based complex that can be used for preparation in a system include triphenylphosphine, tricyclohexylphosphine, tri-t-butylphosphine, 1,2-bis(diphenylphosphino)ethane, 1,3-bis(diphenylphosphino)propane, 1,4-bis(diphenylphosphino)butane, 1,1'-bis(diphenylphosphino)ferrocene, sodium diphenylphosphinobenzene-3-sulfonate, tricyclohexylphosphine, tri(2-furyl)phosphine, tris(2,6-dimethoxyphenyl)phosphine, tris(4-methoxyphenyl)phosphine, tris(4-methylphenyl)phosphine, tris(3-methylphenyl)phosphine, tris(2-methylphenyl)phosphine, and the like.

These palladium catalysts may be used in a range of molar ratio of 0.0001 to 0.1, usually in the range of molar ratio of 0.001 to 0.05, relative to the compound represented by the general formula (III).

As the base that is used in the present invention, inorganic bases and organic bases can be exemplified. Examples of the inorganic bases include alkaline metal hydrides such as lithium hydride, sodium hydride, and potassium hydride; alkaline earth metal hydrides such as magnesium hydride and calcium hydride; alkaline metal hydroxides; alkaline earth metal hydroxides; alkaline metal carbonates; and alkaline earth metal carbonates. Examples of the organic bases include tertiary amines such as trimethylamine, triethylamine, tributylamine, N,N-dimethylaniline, dimethylbenzylamine, and N,N,N',N'-tetramethyl-1,8-naphthalenediamine; heteroaromatic amines such as pyridine, pyrrole, uracil, collidine, and lutidine; cyclic amines such as 1,8-diaza-bicyclo[5.4.0]-7-undecene (DBU), and 1,5-diaza-bicyclo[4.3.0]-5-nonene (DBN); alkaline metal alkoxides such as sodium methoxide, sodium ethoxide, sodium t-butoxide, potassium methoxide, potassium ethoxide, and potassium t-butoxide; alkaline earth metal alkoxides such as magnesium diethoxide and magnesium dimethoxide; and anion exchange resins. These bases may be used singly or in combination of two or more kinds in any ratio, preferably containing either one or more of tertiary amines, heteroaromatic amines and cyclic amidines as a main component.

The used amount of these bases is 0.5 by molar ratio to large excess, preferably 1 to 4 by molar ratio, relative to a hydrocarbon halide represented by the general formula [II].

Preferably, the production method of the present invention is carried out in the presence of an iodine anion generator for the purpose of improving the reaction yield. The iodine anion generator is a compound capable of generating an iodine anion in the reaction system of the present production method, and examples of the iodine anion generator include metal iodides such as lithium iodide, sodium iodide, magnesium iodide, potassium iodide, calcium iodide, cuprous iodide, zinc iodide, iron iodide, and tin iodide; ammonium iodides such as tetraethyl ammonium iodide, tetrabutyl ammonium iodide, benzyltriethyl ammonium iodide, and triethyl ammonium iodide; vinyl iodides such as vinyl iodide and 3,3,3-trifluoro-2-iodopropene; aromatic iodides such as iodobenzene, iodotoluene, iodopyridine, and iodofuran; iodine and hydrogen iodide.

The use amount of these iodine anion generators is 0.5 to 10% by molar ratio, relative to the hydrocarbon halide represented by the general formula [II].

The α-pentafluoroethyl acrylic acid derivative represented by the above formula of the present invention is obtained by letting a hydrocarbon halide represented by the above general formula [II] react with water and/or an alcohol in the presence of a palladium catalyst, carbon monoxide, and a base, however, the reaction may be carried out in the presence of a solvent.

Examples of the solvent include organic bases which are liquid at a reaction temperature among the organic bases as exemplified above; aliphatic hydrocarbons such as pentane, hexane, cyclohexane, heptane, and octane; aromatic hydrocarbons such as benzene, toluene, and xylene; ethers such as diethyl ether, diisopropyl ether, tetrahydrofuran, and dioxane; esters such as ethyl acetate, isopropyl acetate, amyl acetate, butyl acetate, methyl propionate, and ethyl propionate; hydrocarbon halides such as dichloromethane, chloroform, and carbon tetrachloride; ketones such as acetone, methylethyl ketone, methylisobutyl ketone, and cyclohexanone; and polar solvents such as N,N-dimethylformamide, dimethylsulfoxide, 1,3-dimethyl-2-imidazolidinone, acetonitrile, and hexamethyl phosphoric triamide.

The production method of the present invention is carried out at a temperature ranging from room temperature to 300°C, preferably from 60°C to 160°C.

### Example

Next, the present invention will be explained in more detail by way of an example, however, the present invention is not limited to this example.

### Example 1

2-bromo-4,4,4,3,3-pentafluorobutene (31.7 g), triethylamine (28.4 g), dichlorobis(triphenylphosphine)palladium (II) (1.011 g), potassium iodide (0.493 g), water (3.6 g), and tetrahydrofuran (82 g) were loaded into a pressurized vessel, and stirred at a reaction temperature of 80°C, under a carbon monoxide pressure of 0.7 MPaG for 14 hours. Carbon monoxide was continuously supplied using a pressure controller. After cooling, the reaction was allowed to restore to normal pressure, and 6N hydrochloric acid (60 mL) was added and stirred. Then the reaction was separated into two layers, to obtain 72 g of organic layer. A part of the organic layer (10 g) was concentrated, and loaded into a distilling apparatus equipped with a ball. Sequential temperature elevation under a reduced pressure of 3 kPa yielded 0.16 g of fraction at 74°C and 0.60 g of fraction at 88 to 108°C mainly containing α-pentafluoroethyl acrylic acid.
α-pentafluoroethyl acrylic acid
¹H-NMR (200MHz, CDCl₃, δ, ppm):7.027 (1H, t, J = 2.0 Hz), 6.600 (9H, br s)
¹⁹F-NMR (90MHz, CDCl₃, δ, ppm): -83.4 (3F, s), -113.7 (2F, s) GC-MS (EI) m/z:190, 173, 121, 101, 95, 75, 69, 57, 45

### INDUSTRIAL APPLICABILITY

According to the method of the present invention, it is possible to provide an α-pentafluoroethyl acrylic acid derivative which is useful as an intermediate for pharmaceuticals or pesticides and as a functional polymer material, and a method of producing the same.

## Claims

1. An α-pentafluoroethyl acrylic acid derivative represented by the general formula [I]: 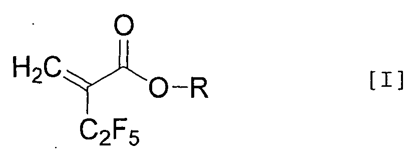
[wherein R represents a hydrogen atom, a non-substituted or substituted aromatic ring, or a straight or branched alkyl group having 1 to 20 carbon(s) which may have a cyclic moiety optionally substituted with at least one substituent (halogen atom, hydroxyl group, straight or branched alkoxy group having 1 to 10 carbon(s) which may have a cyclic moiety, non-substituted or substituted aromatic group)].

2. A method of producing an α-pentafluoroethyl acrylic acid derivative represented by the general formula [I]: 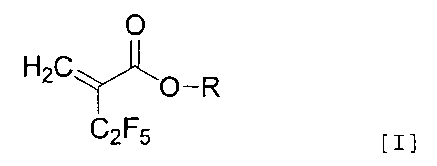
[wherein R represents a hydrogen atom, a non-substituted or substituted aromatic ring, or a straight or branched alkyl group having 1 to 20 carbon(s) which may have a cyclic moiety optionally substituted with at least one substituent (halogen atom, hydroxyl group, straight or branched alkoxy group having 1 to 10 carbon(s) which may have a cyclic moiety, non-substituted or substituted aromatic group)], by letting a hydrocarbon halide represented by the general formula [II]: 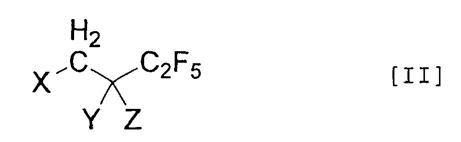
[wherein X represents a halogen atom or forms a bound together with Y, Y represents a hydrogen atom or forms a bond together with X, and Z represents a halogen atom] react with water and/or an alcohol represented by the general formula [III]:
R-OH [III]
[wherein R is as defined above] in the presence of a palladium catalyst, carbon monoxide, and a base.

3. The production method according to claim 2, wherein the reaction is carried out in the presence of an iodine anion generator.
